# EUROPEAN PATENT SPECIFICATION

(11) **EP 3 491 557 B1**
(45) Date of publication and mention of the grant of the patent: **24.04.2024**
(21) Application number: 17742455.3
(22) Date of filing: 27.07.2017
(51) Int. Cl.: G16H 40/63

(54) **PATIENT MONITORING SYSTEM**
PATIENTENÜBERWACHUNGSSYSTEM
SYSTÈME DE SURVEILLANCE DE PATIENTS

(30) Priority: 27.07.2016 EP 16181367
(43) Date of publication of application: 05.06.2019
(73) Proprietor: Koninklijke Philips N.V., 5656 AG Eindhoven (NL)
(72) Inventor: SHAN, Caifeng, 5656 AE Eindhoven (NL); KIRENKO, Ihor, Olehovych, 5656 AE Eindhoven (NL); AARTS, Ronaldus, Maria, 5656 AE Eindhoven (NL); FERTIG, Andreas, 5656 AE Eindhoven (NL)
(74) Representative: Philips Intellectual Property & Standards
(86) International application number: PCT/EP2017/069018
(87) International publication number: WO 2018/019942

(56) References cited:
- WO-A2-2010/102069
- CN-A- 105 204 742
- US-A1- 2005 146 431
- US-A1- 2016 085 922
- Anonymous: "Visible light communication", Wikipedia, 5 July 2016 (2016-07-05), XP055336512, Retrieved from the Internet: URL:https://en.wikipedia.org/w/index.php?t itle=Visible_light_communication&oldid=728 410931 [retrieved on 2017-01-18]
- Anonymous: "Unique Device Identification", Wikipedia, 22 March 2016 (2016-03-22), XP055414239, Retrieved from the Internet: URL:https://en.wikipedia.org/w/index.php?t itle=Unique_Device_Identification&oldid=71 1427065 [retrieved on 2017-10-10]

## Description

### FIELD OF THE INVENTION

The invention relates to a patient monitoring system, method and computer program, wherein the patient monitoring system comprises an output device. The invention relates further to an output device for being used as the output device of the patient monitoring system.

### BACKGROUND OF THE INVENTION

US 2008/0106374 A1 discloses a point of care apparatus comprising an identification system for automatically identifying a role of one or more persons in a room. The point of care apparatus further comprises a processor for determining information relevant to the role of the identified person and a display for displaying the information. The information displayed on the display can be clinical information about a patient, especially if the person is a nurse or a physician.

Further prior art can be found in WO 20101102069 A2, which discloses a bedside patient monitoring system that is adapted to detect the presence of a token device carried by a caregiver in the vicinity of the monitoring device and, in response to the detecting, may adapt the data visualisation and control interface on the display of patient monitoring device based on the identity of the caregiver that is associated with the token device.

### SUMMARY OF THE INVENTION

It is an object of the present invention to provide an improved patient monitoring system, method and computer program, wherein the patient monitoring system comprises an output device. It is a further object of the present invention to provide an improved output device for being used as the output device of the patient monitoring system.

In a first aspect of the present invention a patient monitoring system is presented as defined in claim 1.

Since the output device provides the control interface in accordance with the provided control rules, which in turn are provided depending on the identified user, a user specific control interface for controlling the patient monitoring device can be provided. For instance, the settings of the patient monitoring device can be modified via the control interface provided by the output device, wherein the options of setting the patient monitoring device may depend on the respective user. For example, if the user is a physician, the control interface may allow modifying many settings of the patient monitoring device, and, if the user is a nurse, the control interface may allow a modification of only few settings of the patient monitoring device. This can ensure that the respective user can only modify the respective setting which the user is allowed to modify, thereby reducing the risk of an inappropriate setting of the patient monitoring device. Moreover, by only providing control options, which are relevant for the respective user, the user is less distracted by irrelevant control information.

The patient monitoring device is preferentially adapted to measure vital signs, i.e. the patient monitoring data preferentially include vital signs measurements, and it is preferentially a bedside patient monitoring device. The output device is a mobile device like a tablet computer having a touch sensitive display.

The output device and the patient monitoring device are preferentially adapted to establish a direct data connection. Moreover, the patient monitoring device preferentially comprises a display for showing the patient monitoring data, wherein in an embodiment the patient monitoring device can be adapted to not show the patient monitoring data on the display, if the direct data connection has not been established between the output device and the patient monitoring device. Furthermore, in an embodiment the patient monitoring device can be adapted to show data, which are not the patient monitoring data, if the direct data connection has not been established between the output device and the patient monitoring device. The data, which are not patient monitoring data, can be data that have a relaxing calmative effect like relaxing calmative images or family pictures, in order to set the patient in a positive and healing assisting mood. If a direct data connection has not been established between the output device and the patient monitoring device, it can be assumed that the user, who is preferentially a caregiver, is not close to the patient monitoring device and hence not close to the patient, wherein in this case the data, which are not the patient monitoring data and which might be relaxing calmative data, may be shown on the display of the patient monitoring device. This can lead to an improved healing process of the patient.

The output device comprises a detector with a detection region, wherein the detector is adapted to detect an identity indication of the patient monitoring device, if the identity indication is within the detection region, wherein the patient monitoring device is adapted to provide the identity indication, wherein the output device and the patient monitoring device are adapted such that the output device provides the control interface to the user for controlling the patient monitoring device in accordance with the provided control rules, if the detector detects the identity indication within the detection region. Thus, the output device provides the control interface for controlling the patient monitoring device to the user in accordance with the provided control rules, if the detector detects the identity indication within the detection region. The identity indication can be any indication, which is detectable by the detector, if the identity indication is within the detection region of the detector. Thus, the identity indication is a physical element, which is indicative of the identity of the patient monitoring device and which is detectable by the detector, if the physical element is within the spatial detection region. Different patient monitoring devices might have different identity indications for providing different identities. The detector is preferentially an optical detector like a camera having a field of view defining the detection region and the identity indication is preferentially a visual indication, i.e., for instance, a visual sign or marker, a blinking light, et cetera, wherein the identity indication is indicative of a respective patient monitoring device. Thus, identity information can be encoded in the identity indication. If several patient monitoring devices are in a room, wherein each patient monitoring device comprises a respective identity indication, it can be selected to which patient monitoring device the output device should be connected, in order to allow the output device to provide a control interface to the user for controlling the selected patient monitoring device, by holding the output device such that the identity indication of the patient monitoring device to be selected is within the detection region. This allows the user to relatively easily select the patient monitoring device, which should be connected to the output device, by just holding the output device accordingly.

The output device and the patient monitoring device can be adapted to establish a data connection by using at least one of visible light communication (VLC) and near field communication (NFC). Using VLC or NFC for establishing the data connection allows for a reliable and fast direct data connection, thereby further improving the patient monitoring system.

The data connection between the output device and the patient monitoring device is preferentially a two-way data connection. This can allow for a fully functional remote access to the patient monitoring device via the output device.

In an embodiment the output device and the patient monitoring device are adapted to establish a data connection via a data network. The data network is, for instance, a wired or wireless local area network. This allows controlling the patient monitoring device via the output device, even if, for instance, the user holding the output device is not in a patient room where the patient and the patient monitoring device might be located, but in another room. Thus, a user specific remote control for the patient monitoring device can be provided, which allows controlling the patient monitoring device, even if the user is in another room not being the room in which the patient is located.

The output device is preferentially adapted to provide a user interface for allowing the user to input user specific data, wherein the user identification unit is adapted to identify the user based on the input user specific data. Thus, it is not required, for instance, to use radio-frequency identification (RFID) techniques, but it is just required that the user inputs user specific data like his name and a password, in order to identify the user. The input user specific data can also be biometric data like a finger print, retina blood vessels, et cetera.

It is preferred that the rules providing unit is also adapted to provide visualization rules, which define a provision of a visualization of the patient monitoring data, depending on the identified user, wherein the output device is adapted to provide a visualization of the patient monitoring data in accordance with the provided visualization rules. Thus, the patient monitoring system can also be adapted to provide user specific patient monitoring data. For instance, more patient monitoring data may be shown to a physician than to a nurse. By this more focused visualization of the patient monitoring data the user might be less distracted by irrelevant patient monitoring data, thereby further improving the patient monitoring system.

In a further aspect of the present invention a patient monitoring method is presented as defined in claim 13.

In a further aspect of the present invention an output device for being used as the output device of the patient monitoring system as defined in claim 1 is presented as defined in claim 12.

Thus, in this aspect of the present invention the output device, which is adapted to be used as the output device of the patient monitoring system of claim 1, includes the user identification unit and the rules providing unit.

In another aspect of the present invention a computer program for controlling the patient monitoring system as defined in claim 1 is presented, wherein the computer program comprises program code means for causing the patient monitoring system as defined in claim 1 to carry out the patient monitoring method as defined in claim 13, when the computer program is run on the patient monitoring system.

It shall be understood that patient monitoring system of claim 1, the output device of claim 12, the patient monitoring method of claim 13 and the computer program for controlling the patient monitoring system of claim 14 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a patient monitoring system,
Fig. 2 shows schematically and exemplarily an embodiment of an output device of the patient monitoring system, and
Fig. 3 shows a flowchart exemplarily illustrating an embodiment of a patient monitoring method.

### DETAILED DESCRIPTION OF EMBODIMENTS

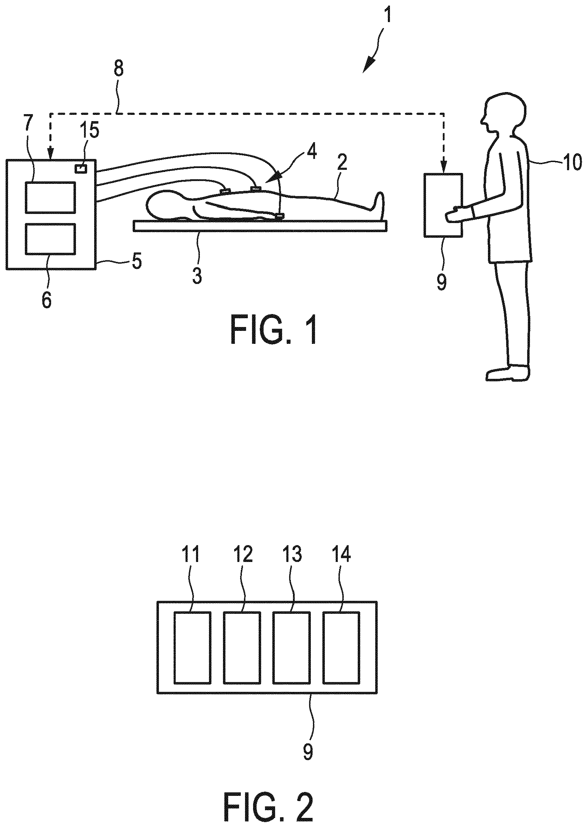

An embodiment of a patient monitoring system 1 is schematically and exemplarily illustrated in Fig. 1. The patient monitoring system 1 comprises a patient monitoring device 5 for monitoring a patient 2 lying on a patient bed 3, wherein the patient monitoring device 5 is adapted to generate patient monitoring data. In particular, the patient monitoring device 5 comprises a measuring unit 6 connected to sensors 4 attached to the patient 2, wherein the measuring unit 6 and the sensors 4 are adapted to measure vital signs, in order to monitor the patient 2 and generate the patient monitoring data. For instance, the measuring unit 6 and the sensors 4 might be adapted to measure the heart rate, the respiratory rate, the blood oxygen saturation level, the body temperature, the blood pressure, et cetera. The patient monitoring device 5 further comprises a display 7 for showing at least a part of the patient monitoring data and for also showing data, which are not patient monitoring data, like relaxing calmative images or family pictures, in order to set the patient in a positive and healing assisting mood.

The patient monitoring system 1 further comprises an output device 9 for providing a control interface for controlling the patient monitoring device 5. In this embodiment the patient monitoring device 5 is a bedside device and the output device 9 is a mobile tablet computer having a touch sensitive display for providing the control interface and for receiving inputs from a user 10 being preferentially a caregiver like a nurse or a physician. The output device 9 is further adapted to provide a visualization of the patient monitoring data. Thus, via the output device 9 the patient monitoring device 5 can be controlled and patient monitoring data can be shown to the user 10.

The output device 9 is schematically and exemplarily illustrated in Fig. 2. The output device 9 comprises a user identification unit 11 for identifying the user 10 and a rules providing unit 12 for providing control rules, which define a provision of a control interface for controlling the patient monitoring device 5, depending on the identified user 10. In this embodiment the rules providing unit 12 is also adapted to provide visualization rules, which define a provision of a visualization of the patient monitoring data, depending on the identified user 10. The output device 9 is adapted to provide the control interface for controlling the patient monitoring device in accordance with the provided control rules and to provide the visualization of the patient monitoring data in accordance with the provided visualization rules. The visualization rules preferentially define which of the patient monitoring data should be shown on the output device 9, wherein the selection of the patient monitoring data to be shown depends on the identified user 10. The control rules preferentially define which settings of the patient monitoring device the user 10 is allowed to modify, i.e. the control rules can define a selection of controls usable by the user 10.

The output device 9 comprises a touch sensitive display 13 for showing information and for receiving inputs. It should be noted that Fig. 2 is of course only a schematic figure, i.e. the touch sensitive display 13 preferentially covers an entire side of the output device 9, and the user identification unit 11, the rules providing unit 12 and a detector 14, which will be described below, are integrated within a casing of the output device 9.

In this embodiment the output device 9 and the patient monitoring device 5 are adapted to establish a direct data connection 8, wherein the direct data connection 8 is preferentially established by using a VLC or a NFC technique. The direct data connection 8 is a two-way data connection such that, for instance, patient monitoring data can be sent from the patient monitoring device 5 to the output device 9 and control information for controlling the patient monitoring device 5 can be sent from the output device 9 to the patient monitoring device 5.

The patient monitoring device 5 comprises an identity indication 15 being indicative of the identity of the patient monitoring device 5. The identity indication 15 is an optically detectable marker. This optically detectable marker can be a sequence of signs like a sequence of numbers and/or letters, a barcode, et cetera. The output device 9 comprises a detector 14 with a detection region, wherein the detector 14 is adapted to detect the identity indication 15 of the patient monitoring device 5, if the identity indication 15 is within the detection region. In this embodiment the detector 14 is a camera of the tablet computer 9, wherein the detection region is the field of view of the camera 14. The tablet computer 9 and the patient monitoring device 5 are adapted such that the data connection 8 is established between the tablet computer 9 and the patient monitoring device 5, if the identity indication 15 is within the field of view of the camera 14. Thus, the tablet computer 9 is adapted to detect the identity indication 15 within an image acquired by the camera 14, wherein, if the identity indication 15 has been detected in the image, the data connection 8 is established. If several patient monitoring devices are within a same patient room, the user 10 can select a desired patient monitoring device to be connected to the tablet computer 9 by pointing the tablet computer 9 to the patient monitoring device to be selected. If several identity indications of several patient monitoring devices are within the detection region of the detector of the output device, the output device may provide a graphical user interface indicating the patient monitoring devices with the identity indications within the detection region and allowing the user to select one of these patient monitoring devices. The data connection can then be established between the selected patient monitoring device and the output device.

The patient monitoring device 5 can be adapted to show data, which are not the patient monitoring data, like relaxing calmative images or family pictures, if the direct data connection 8 has not been established between the output device 9 and the patient monitoring device 5. Thus, if a caregiver carrying the output device 9 is not in the vicinity of the patient 2, the patient monitoring device 5 may automatically show the relaxing calmative information on its display 7.

The output device 9 is preferentially adapted to provide a user interface for allowing the user 10 to input user specific data, wherein the user identification unit 11 is adapted to identify the user 10 based on the input user specific data. The user specific data can be, for instance, login data like a user name and a password, wherein the user identification unit 11 can be adapted to identify the user based on the user name and the password. The user specific data can also be other kind of data like biometric data.

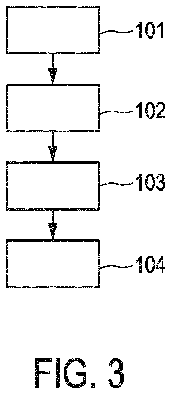

In the following an embodiment of a patient monitoring method will exemplarily be described with reference to a flowchart shown in Fig. 3.

While the patient monitoring device 5 continuously monitors the patient 2, in order to generate patient monitoring data, in step 101 the user 10 is identified by the user identification unit 11. In step 102 control rules, which define a provision of a control interface for controlling the patient monitoring device 5, are provided depending on the identified user 10 by the rules providing unit 12. Moreover, visualization rules, which define a provision of a visualization of the patient monitoring data, are provided depending on the identified user 10 by the rules providing unit 12. In step 103 the output device 9 provides a control interface for controlling the patient monitoring device in accordance with the provided control rules and a visualization of the patient monitoring data in accordance with the provided visualization rules. In step 104 the patient monitoring device 5 is controlled depending on control information input by the user 10 via the provided control interface. Steps 101 and 102 can be exchanged, i.e. it is also possible that firstly step 102 is carried out and then step 101.

Although in above described embodiments VLC or NFC are used for establishing a data connection between the output device and the patient monitoring device, the data connection can also be established by using another data connection technique like Bluetooth.

Although in above described embodiments a direct data connection is established between the patient monitoring device and the output device via NFC or VLC, the output device and the patient monitoring device may also be adapted to establish a data connection via a data network like a wired or wireless local area network. This can allow for a control of the patient monitoring device and a provision of patient monitoring data via the output device, even if the user with the output device is not in a patient room in which the patient monitoring device is located.

In medicine monitoring can be regarded as being the observation of a disease, condition or one or several medical parameters over time. It can be performed by continuously measuring certain parameters by using a medical monitor, i.e. a patient monitoring device which may be adapted to continuously measure vital signs at a bedside. If a patient monitoring device always shows substantially the same fixed patient monitoring data to all kinds of users, this is not always useful, because different kinds of users like a doctor, a specialist, a nurse practitioner, a nurse, a patient et cetera have different needs of displaying patient monitoring data like different parameters, more details or less details, et cetera and need different levels of control of the patient monitoring device. The patient monitoring system and method described above can therefore allow a caregiver to see what he/she likes to see on a display and where he/she likes to see the respective information on the display, wherein this is not necessarily in the vicinity of the patient and hence this information is shown on the additional display of the output device. Depending on the person holding and watching at the output device, different patient monitoring data from the patient monitoring device can be shown on the display of the output device. Also a user dependent level of control of the patient monitoring device is provided. Thus, one source of patient monitoring data can be related to different visualizations, i.e. especially visibilities, and different controls depending on the respective user. This allows for, for instance, a caregiver centric display instead of a patient centric display.

Preferentially the patient monitoring device is located next to a patient and collects many vital signs of the patient, but displays only limited information on a small display of the patient monitoring device, wherein this limited information may include the blood oxygen saturation level, the heart rate, the respiratory rate and an alarm which is especially initiated, if a measured value is not within expected acceptable predefined ranges. Every caregiver has its own monitor, i.e. output device like a tablet computer, which is used to extract the information from the patient monitoring device, which is needed for the respective specific caregiver. For instance, to a nurse only limited information might be shown, whereas to a doctor also a history of previous measurements and additional information might be provided. Each caregiver can have different controls on its own monitor, for instance, in order to set different alerts at different levels at the patient monitoring device. Based on the respective person, i.e., for example, nurse or physician, holding the monitor different types of patient monitoring data like vital signs, electrocardiogram data, et cetera at different detail level are visible on the monitor.

By using only one source of patient data only, i.e. the patient monitoring device, but different visualizations and controls, which depend on the respective user, privacy issued can be solved. Moreover, the data connection between the patient monitoring device and the output device is preferentially a secure data connection such that the transferred data are accessible only for the intended persons.

The patient monitoring device can be adapted to show some kind of standby screen, if a caregiver like a nurse or physician is not in the vicinity of the patient monitoring device, in order to hide privacy related data. The empty screen can be filled with data relevant to the patient. For example, if the patient monitoring device is used in a children station, the display of the patient monitoring device can show relaxing calmative images or family pictures to set a positive and healing assisting mood. If a caregiver enters the patient room, the display of the patient monitoring device can be activated such that it shows patient monitoring data, wherein this entering of the room can be detected by the establishing of the data connection between the mobile output device carried by the caregiver and the patient monitoring device and wherein then the display of the patient monitoring device and/or the display of the output device may get a customized screen.

Although in above described embodiments the output device is a tablet computer, in other embodiments it can be another output device like a wearable device, for instance, an optical head-mounted display device.

Although in above described embodiments the user identification unit and the rules providing unit are integrated in the output device, in other embodiments not falling in the scope of the claims one of these units or both units can also be integrated in the patient monitoring device or in another device to which the output device and optionally also the patient monitoring device might be connected via a data connection.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

The control of the patient monitoring system in accordance with the patient monitoring method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a patient monitoring system comprising a patient monitoring device for monitoring a patient, wherein the patient monitoring device is adapted to generate patient monitoring data. An output device provides a control interface, which is adapted to allow the user to control the patient monitoring device, in accordance with provided user-specific control rules. For instance, the settings of the patient monitoring device can be modified via the provided control interface, wherein the options of setting the patient monitoring device can depend on the respective user. This can ensure that the respective user can only modify the respective setting which the user is allowed to modify, thereby reducing the risk of inappropriate settings of the patient monitoring device. Moreover, by only providing control options, which are relevant for the respective user, the user can be less distracted by irrelevant control information.

## Claims

1. A patient monitoring system (1) comprising:
- a patient monitoring device (5) adapted to monitor a patient (2), thereby generating patient monitoring data, and
- an output device (9) adapted to provide a user-specific control interface to a user (10), which is adapted to allow the user to control the patient monitoring device (5), in accordance with user-specific control rules, wherein the output device (9) is a mobile device which is separated from the patient monitoring device (5) and which comprises:
- a user identification unit (11) adapted to identify the user (10),
- a rules providing unit (12) adapted to provide the user-specific control rules, which define a provision of the user-specific control interface for controlling the patient monitoring device (5), depending on the identified user (10),
- a detector (14) with a detection region, wherein the detector is adapted to detect an identity indication (15) of the patient monitoring device (5) indicative of the identity of the patient monitoring device, if the identity indication (15) is within the detection region,
wherein the patient monitoring device (5) is adapted to provide the identity indication (15), and
wherein the output device (9) and the patient monitoring device (5) are adapted such that the output device (9) provides the user-specific control interface to the user (10) for controlling the patient monitoring device (5) in accordance with the provided user-specific control rules, if the detector (14) detects the identity indication (15) within the detection region.

2. The patient monitoring system as defined in claim 1, wherein the output device (9) and the patient monitoring device (5) are adapted to establish a direct data connection (8).

3. The patient monitoring system as defined in claim 2, wherein the patient monitoring device (5) comprises a display (7) adapted to show the patient monitoring data, wherein the patient monitoring device (5) is adapted to not show the patient monitoring data on the display (7), if the direct data connection (8) has not been established between the output device (9) and the patient monitoring device (5).

4. The patient monitoring system as defined in claim 2, wherein the patient monitoring device (5) comprises a display (7) and is adapted to show data on the display (7), which are not the patient monitoring data, if the direct data connection (8) has not been established between the output device (9) and the patient monitoring device (5).

5. The patient monitoring system as defined in claim 1, wherein the detector (14) is an optical detector having a field of view defining the detection region and the identity indication (15) is a visual indication.

6. The patient monitoring system as defined in claim 1, wherein the output device (9) and the patient monitoring device (5) are adapted to establish a data connection by using at least one of visible light communication (VLC) and near field communication (NFC).

7. The patient monitoring system as defined in claim 1, wherein the output device (9) and the patient monitoring device (5) are adapted to establish a two-way data connection.

8. The patient monitoring system as defined in claim 1, wherein the output device (9) and the patient monitoring device (5) are adapted to establish a data connection via a data network.

9. The patient monitoring system as defined in claim 1, wherein the output device (9) is adapted to provide a user interface for allowing the user (10) to input user specific data, wherein the user identification unit (11) is adapted to identify the user (10) based on the input user specific data.

10. The patient monitoring system as defined in claim 1, wherein the mobile device is a tablet computer.

11. The patient monitoring system as defined in claim 1, wherein the rules providing unit (12) is adapted to provide visualization rules, which define a provision of a visualization of the patient monitoring data, depending on the identified user (10), wherein the output device (9) is adapted to provide a visualization of the patient monitoring data in accordance with the provided visualization rules.

12. An output device for being used as the output device (9) of the patient monitoring system as defined in claim 1, wherein the output device (9) comprises:
- the user identification unit (11) adapted to identify a user (10),
- the rules providing unit (12) of the patient monitoring system adapted to provide user-specific control rules, which define the provision of a user-specific control interface for controlling the patient monitoring device (5) of the patient monitoring system, depending on the identified user (10), and
- the detector (14) with the detection region, wherein the detector (14) is adapted to detect identity indication (15) of the patient monitoring device (5) indicative of the identity of the patient monitoring device, if the identity indication (15) is within the detection region,
wherein the output device (9) is adapted to provide a control interface to a user (10), which is adapted to allow the user to control the patient monitoring device (5), in accordance with the provided user-specific control rules,
wherein the output device (9) is a mobile device which is separated from the patient monitoring device (5), and
wherein the output device (9) is adapted such that the output device (9) provides the user-specific control interface to the user (10) for controlling the patient monitoring device (5) in accordance with the provided user-specific control rules, if the detector (14) detects the identity indication (15) within the detection region.

13. A patient monitoring method comprising:
- monitoring a patient (2) by a patient monitoring device (5), thereby generating patient monitoring data,
- identifying a user (10) by a user identification unit (11),
- providing user-specific control rules, which define a provision of a user-specific control interface for controlling the patient monitoring device (5), depending on the identified user (10) by a rules providing unit (12),
- providing the user-specific control interface to the user (10), which is adapted to allow the user to control the patient monitoring device (5), in accordance with the provided user-specific control rules by an output device (9) being a mobile device which is separated from the patient monitoring device (5) and comprising a detector (14) with a detection region,
wherein the detector (14) detects an identity indication (15) of the patient monitoring device (5) indicative of the identity of the patient monitoring device, if the identity indication (15) is within the detection region,
wherein the patient monitoring device (5) provides the identity indication (15), wherein the output device (9) provides the user-specific control interface to the user (10) for controlling the patient monitoring device (5) in accordance with the provided user-specific control rules, if the detector (14) detects the identity indication (15) within the detection region.

14. A computer program for controlling the patient monitoring system as defined in claim 1, the computer program comprising program code means for causing the patient monitoring system as defined in claim 1 to carry out the patient monitoring method as defined in claim 13, when the computer program is run on the patient monitoring system.

## Patentansprüche

1. Patientenüberwachungssystem (1), umfassend:
- eine Patientenüberwachungsvorrichtung (5), die angepasst ist, um einen Patienten (2) zu überwachen, wodurch Patientenüberwachungsdaten erzeugt werden, und
- eine Ausgabevorrichtung (9), die angepasst ist, um einem Benutzer (10) eine benutzerspezifische Steuerschnittstelle bereitzustellen, die angepasst ist, um dem Benutzer zu ermöglichen, die Patientenüberwachungsvorrichtung (5) gemäß benutzerspezifischen Steuerungsregeln zu steuern, wobei die Ausgabevorrichtung (9) ist, die von der Patientenüberwachungsvorrichtung (5) getrennt ist, und die Folgendes umfasst:
- eine Benutzeridentifizierungseinheit (11), die angepasst ist, um den Benutzer (10) zu identifizieren,
- eine Regelbereitstellungseinheit (12), die angepasst ist, um die benutzerspezifischen Steuerungsregeln bereitzustellen, die eine Bereitstellung der benutzerspezifischen Steuerungsschnittstelle zum Steuern der Patientenüberwachungsvorrichtung (5) abhängig von dem identifizierten Benutzer (10) zu definieren,
- einen Detektor (14) mit einem Erfassungsbereich, wobei der Detektor angepasst ist, um eine Identitätsangabe (15) der Patientenüberwachungsvorrichtung (5) zu erfassen, die indikativ für die Identität der Patientenüberwachungsvorrichtung ist, wenn die Identitätsangabe (15) innerhalb des Erfassungsbereichs ist, wobei die Patientenüberwachungsvorrichtung (5) angepasst ist, um die Identitätsangabe (15) bereitzustellen, und wobei die Ausgabevorrichtung (9) und die Patientenüberwachungsvorrichtung (5) angepasst sind, sodass die Ausgabevorrichtung (9) dem Benutzer (10) die benutzerspezifische Steuerschnittstelle zum Steuern der Patientenüberwachungsvorrichtung (5) gemäß den bereitgestellten benutzerspezifischen Steuerregeln bereitstellt, wenn der Detektor (14) die Identitätsangabe (15) innerhalb des Erfassungsbereichs erfasst.

2. Patientenüberwachungssystem nach Anspruch 1, wobei die Ausgabevorrichtung (9) und die Patientenüberwachungsvorrichtung (5) angepasst sind, um eine direkte Datenverbindung (8) herzustellen.

3. Patientenüberwachungssystem nach Anspruch 2, wobei die Patientenüberwachungsvorrichtung (5) eine Anzeige (7) umfasst, die angepasst ist, um die Patientenüberwachungsdaten anzuzeigen, wobei die Patientenüberwachungsvorrichtung (5) angepasst ist, um die Patientenüberwachungsdaten nicht auf der Anzeige (7) anzuzeigen, wenn die direkte Datenverbindung (8) zwischen der Ausgabevorrichtung (9) und der Patientenüberwachungsvorrichtung (5) nicht hergestellt worden ist.

4. Patientenüberwachungssystem nach Anspruch 2, wobei die Patientenüberwachungsvorrichtung (5) eine Anzeige (7) umfasst und angepasst ist, um auf der Anzeige (7) Daten anzuzeigen, die nicht die Patientenüberwachungsdaten sind, wenn die direkte Datenverbindung (8) zwischen der Ausgabevorrichtung (9) und der Patientenüberwachungsvorrichtung (5) nicht hergestellt worden ist.

5. Patientenüberwachungssystem nach Anspruch 1, wobei der Detektor (14) ein optischer Detektor ist, der ein Sichtfeld aufweist, das den Erfassungsbereich definiert, und die Identitätsangabe (15) eine visuelle Angabe ist.

6. Patientenüberwachungssystem nach Anspruch 1, wobei die Ausgabevorrichtung (9) und die Patientenüberwachungsvorrichtung (5) angepasst sind, um unter Verwendung von Visible Light Communication (VLC) und/oder Nahfeldkommunikation (NFC) eine Datenverbindung herzustellen.

7. Patientenüberwachungssystem nach Anspruch 1, wobei die Ausgabevorrichtung (9) und die Patientenüberwachungsvorrichtung (5) angepasst sind, um eine bidirektionale Verbindung herzustellen.

8. Patientenüberwachungssystem nach Anspruch 1, wobei die Ausgabevorrichtung (9) und die Patientenüberwachungsvorrichtung (5) angepasst sind, um eine Datenverbindung über ein Datennetzwerk herzustellen.

9. Patientenüberwachungssystem nach Anspruch 1, wobei die Ausgabevorrichtung (9) angepasst ist, um eine Benutzerschnittstelle bereitzustellen, die es dem Benutzer (10) ermöglicht, benutzerspezifische Daten einzugeben, wobei die Benutzeridentifizierungseinheit (11) angepasst ist, um den Benutzer (10) basierend auf den eingegebenen benutzerspezifischen Daten zu identifizieren.

10. Patientenüberwachungssystem nach Anspruch 1, wobei die mobile Vorrichtung ein Tablet-Computer ist.

11. Patientenüberwachungssystem nach Anspruch 1, wobei die Regelbereitstellungseinheit (12) angepasst ist, um Visualisierungsregeln bereitzustellen, die eine Bereitstellung einer Visualisierung der Patientenüberwachungsdaten abhängig von dem identifizierten Benutzer (10) zu definieren, wobei die Ausgabevorrichtung (9) angepasst ist, um eine Visualisierung der Patientenüberwachungsdaten in Übereinstimmung mit den bereitgestellten Visualisierungsregeln bereitzustellen.

12. Ausgabevorrichtung zur Verwendung als Ausgabevorrichtung (9) des Patientenüberwachungssystem nach Anspruch 1, wobei die Ausgabevorrichtung (9) Folgendes umfasst:
- die Benutzeridentifizierungseinheit (11), die angepasst ist, um einen Benutzer (10) zu identifizieren,
- die Regelbereitstellungseinheit (12) des Patientenüberwachungssystems, die angepasst ist, um benutzerspezifische Steuerungsregeln bereitzustellen, die die Bereitstellung einer benutzerspezifischen Steuerungsschnittstelle zum Steuern der Patientenüberwachungsvorrichtung (5) des Patientenüberwachungssystems abhängig von dem identifizierten Benutzer (10) zu definieren, und
- den Detektor (14) mit dem Erfassungsbereich, wobei der Detektor (14) angepasst ist, um eine Identitätsangabe (15) der Patientenüberwachungsvorrichtung (5) zu erfassen, die indikativ für die Identität der Patientenüberwachungsvorrichtung ist, wenn die Identitätsangabe (15) innerhalb des Erfassungsbereichs ist, wobei die Ausgabevorrichtung (9) angepasst ist, um einem Benutzer (10) eine Steuerschnittstelle bereitzustellen, die angepasst ist, um dem Benutzer zu ermöglichen, die Patientenüberwachungsvorrichtung (5) in Übereinstimmung mit den bereitgestellten benutzerspezifischen Steuerregeln zu steuern, wobei die Ausgabevorrichtung (9) eine mobile Vorrichtung ist, die von der Patientenüberwachungsvorrichtung (5) getrennt ist, und wobei die Ausgabevorrichtung (9) angepasst ist, sodass die Ausgabevorrichtung (9) dem Benutzer (10) die benutzerspezifische Steuerschnittstelle zum Steuern des Patientenüberwachungsvorrichtung (5) gemäß den bereitgestellten benutzerspezifischen Steuerregeln bereitstellt, wenn der Detektor (14) die Identitätsangabe (15) innerhalb des Erfassungsbereichs erfasst.

13. Patientenüberwachungsverfahren, umfassend:
- Überwachen eines Patienten (2) durch eine Patientenüberwachungsvorrichtung (5), wodurch Patientenüberwachungsdaten erzeugt werden,
- Identifizieren eines Benutzers (10) durch eine Benutzeridentifizierungseinheit (11),
- Bereitstellen von benutzerspezifischen Steuerungsregeln, die eine Bereitstellung einer benutzerspezifischen Steuerungsschnittstelle zum Steuern der Patientenüberwachungsvorrichtung (5) abhängig von dem identifizierten Benutzer (10) durch eine Regelbereitstellungseinheit (12) definieren,
- Bereitstellen der benutzerspezifischen Steuerschnittstelle für den Benutzer (10), die angepasst ist, um dem Benutzer zu ermöglichen, die Patientenüberwachungsvorrichtung (5) gemäß den bereitgestellten benutzerspezifischen Steuerregeln durch eine Ausgabevorrichtung (9) zu steuern, die eine mobile Vorrichtung ist, die von der Patientenüberwachungsvorrichtung (5) getrennt ist und einen Detektor (14) mit einem Erfassungsbereich umfasst, wobei der Detektor (14) eine Identitätsangabe (15) der Patientenüberwachungsvorrichtung (5) erfasst, die die Identität der Patientenüberwachungsvorrichtung angibt, wenn die Identitätsangabe (15) innerhalb des Erfassungsbereichs ist, wobei die Patientenüberwachungsvorrichtung (5) die Identitätsangabe (15) bereitstellt, wobei die Ausgabevorrichtung (9) dem Benutzer (10) die benutzerspezifische Steuerschnittstelle zum Steuern der Patientenüberwachungsvorrichtung (5) gemäß den bereitgestellten benutzerspezifischen Steuerregeln bereitstellt, wenn der Detektor (14) die Identitätsangabe (15) innerhalb des Erfassungsbereichs erkennt.

14. Computerprogramm zum Steuern des Patientenüberwachungssystems nach Anspruch 1, das Computerprogramm umfassend Programmcodeeinrichtungen, um das Patientenüberwachungssystem nach Anspruch 1 zu veranlassen, das Patientenüberwachungsverfahren nach Anspruch 13 auszuführen, wenn das Computerprogramm auf dem Patientenüberwachungssystem ausgeführt wird.

## Revendications

1. Système de surveillance du patient (1) comprenant:
- un dispositif de surveillance du patient (5) adapté à la surveillance d'un patient (2), générant ainsi des données de surveillance du patient, et
- un dispositif de sortie (9) adapté pour fournir une interface de commande spécifique à un utilisateur (10), qui est adapté pour permettre à l'utilisateur de commander le dispositif de surveillance du patient (5), conformément aux règles de commande spécifiques à l'utilisateur, dans lequel le dispositif de sortie (9) est un dispositif mobile qui est séparé du dispositif de surveillance du patient (5) et qui comprend:
- une unité d'identification de l'utilisateur (11) permettant d'identifier l'utilisateur (10),
- une unité de fourniture de règles (12) adaptée pour fournir les règles de commande spécifiques à l'utilisateur, qui définissent la fourniture de l'interface de commande spécifique à l'utilisateur pour commander le dispositif de surveillance du patient (5), en fonction de l'utilisateur identifié (10),
- un détecteur (14) doté d'une zone de détection, dans lequel le détecteur est adapté pour détecter une indication d'identité (15) du dispositif de surveillance du patient (5) indiquant l'identité du dispositif de surveillance du patient, si l'indication d'identité (15) se trouve à l'intérieur de la zone de détection, dans lequel le dispositif de surveillance du patient (5) est adapté pour fournir l'indication d'identité (15), et dans lequel le dispositif de sortie (9) et le dispositif de surveillance du patient (5) sont adaptés pour que le dispositif de sortie (9) fournisse l'interface de commande spécifique à l'utilisateur à l'utilisateur (10) pour commander le dispositif de surveillance du patient (5) conformément aux règles de commande spécifiques à l'utilisateur fournies, si le détecteur (14) détecte l'indication d'identité (15) dans la zone de détection.

2. Système de surveillance du patient tel que défini dans la revendication 1, dans lequel le dispositif de sortie (9) et le dispositif de surveillance du patient (5) sont adaptés pour établir une connexion de données directe (8).

3. Système de surveillance du patient tel que défini dans la revendication 2, dans lequel le dispositif de surveillance du patient (5) comprend un écran (7) adapté pour afficher les données de surveillance du patient, dans lequel le dispositif de surveillance du patient (5) est adapté pour ne pas afficher les données de surveillance du patient sur l'écran (7), si la connexion directe de données (8) n'a pas été établie entre le dispositif de sortie (9) et le dispositif de surveillance du patient (5).

4. Système de surveillance du patient tel que défini dans la revendication 2, dans lequel le dispositif de surveillance du patient (5) comprend un écran (7) et est adapté pour afficher des données sur l'écran (7), qui ne sont pas les données de surveillance du patient, si la connexion directe de données (8) n'a pas été établie entre le dispositif de sortie (9) et le dispositif de surveillance du patient (5).

5. Système de surveillance du patient tel que défini dans la revendication 1, dans lequel le détecteur (14) est un détecteur optique ayant un champ de vision définissant la région de détection et l'indication d'identité (15) est une indication visuelle.

6. Système de surveillance du patient tel que défini dans la revendication 1, dans lequel le dispositif de sortie (9) et le dispositif de surveillance du patient (5) sont adaptés pour établir une connexion de données en utilisant au moins l'une des communications par lumière visible (VLC) et la communication en champ proche (NFC).

7. Système de surveillance du patient tel que défini dans la revendication 1, dans lequel le dispositif de sortie (9) et le dispositif de surveillance du patient (5) sont adaptés pour établir une connexion de données bidirectionnelle.

8. Système de surveillance du patient tel que défini dans la revendication 1, dans lequel le dispositif de sortie (9) et le dispositif de surveillance du patient (5) sont adaptés pour établir une connexion de données via un réseau de données.

9. Système de surveillance du patient tel que défini dans la revendication 1, dans lequel le dispositif de sortie (9) est adapté pour fournir une interface utilisateur permettant à l'utilisateur (10) d'entrer des données spécifiques à l'utilisateur, dans lequel l'unité d'identification de l'utilisateur (11) est adaptée pour identifier l'utilisateur (10) sur la base des données spécifiques à l'utilisateur entrées.

10. Système de surveillance du patient tel que défini dans la revendication 1, dans lequel le dispositif mobile est un ordinateur tablette.

11. Système de surveillance du patient tel que défini dans la revendication 1, dans lequel l'unité de fourniture de règles (12) est adaptée pour fournir des règles de visualisation, qui définissent la fourniture d'une visualisation des données de surveillance du patient, en fonction de l'utilisateur identifié (10), dans lequel le dispositif de sortie (9) est adapté pour fournir une visualisation des données de surveillance du patient en conformité avec les règles de visualisation fournies.

12. Dispositif de sortie destiné à être utilisé comme dispositif de sortie (9) du système de surveillance du patient tel que défini dans la revendication 1, dans lequel le dispositif de sortie (9) comprend:
- l'unité d'identification de l'utilisateur (11) adaptée pour identifier un utilisateur (10),
- l'unité de fourniture de règles (12) du système de surveillance du patient adaptée pour fournir des règles de commande spécifiques à l'utilisateur, qui définissent la fourniture d'une interface de commande spécifique à l'utilisateur pour commander le dispositif de surveillance du patient (5) du système de surveillance du patient, en fonction de l'utilisateur identifié (10), et
- le détecteur (14) avec la zone de détection, le détecteur (14) étant adapté pour détecter l'indication d'identité (15) du dispositif de surveillance du patient (5) indiquant l'identité du dispositif de surveillance du patient, si l'indication d'identité (15) se trouve dans la zone de détection, dans lequel le dispositif de sortie (9) est adapté pour fournir une interface de commande à un utilisateur (10), qui est adapté pour permettre à l'utilisateur de commander le dispositif de surveillance du patient (5), conformément aux règles de commande spécifiques à l'utilisateur fournies, dans lequel le dispositif de sortie (9) est un dispositif mobile qui est séparé du dispositif de surveillance du patient (5), et dans lequel le dispositif de sortie (9) est adapté de manière à ce que le dispositif de sortie (9) pour fournir à l'utilisateur (10) l'interface de commande spécifique à l'utilisateur pour commander le dispositif de surveillance du patient (5) conformément aux règles de commande spécifiques à l'utilisateur fournies, si le détecteur (14) détecte l'indication d'identité (15) dans la zone de détection.

13. Méthode de surveillance d'un patient comprenant les étapes consistant à:
- surveiller un patient (2) au moyen d'un dispositif de surveillance du patient (5), ce qui permet de générer des données de surveillance du patient,
- identifier un utilisateur (10) au moyen d'une unité d'identification de l'utilisateur (11),
- fournir des règles de commande spécifiques à l'utilisateur, qui définissent la fourniture d'une interface de commande spécifique à l'utilisateur pour commander le dispositif de surveillance du patient (5), en fonction de l'utilisateur identifié (10) par une unité de fourniture de règles (12),
- fournir à l'utilisateur (10) l'interface de commande spécifique à l'utilisateur, qui est adaptée pour permettre à l'utilisateur de commander le dispositif de surveillance du patient (5), conformément aux règles de commande spécifiques à l'utilisateur fournies par un dispositif de sortie (9) qui est un dispositif mobile séparé du dispositif de surveillance du patient (5) et qui comprend un détecteur (14) avec une zone de détection, dans lequel le détecteur (14) détecte une indication d'identité (15) du dispositif de surveillance du patient (5) indiquant l'identité du dispositif de surveillance du patient, si l'indication d'identité (15) se trouve dans la zone de détection, dans lequel le dispositif de surveillance du patient (5) fournit l'indication d'identité (15), le dispositif de sortie (9) fournit l'interface de commande spécifique à l'utilisateur (10) pour commander le dispositif de surveillance du patient (5) conformément aux règles de commande spécifiques à l'utilisateur fournies, si le détecteur (14) détecte l'indication d'identité (15) dans la zone de détection.

14. Programme informatique pour commander le système de surveillance du patient tel que défini dans la revendication 1, le programme d'ordinateur comprenant des moyens de code de programme pour amener le système de surveillance du patient tel que défini dans la revendication 1 à exécuter la méthode de surveillance du patient telle que définie dans la revendication 13, lorsque le programme d'ordinateur est exécuté sur le système de surveillance du patient.
